# EUROPEAN PATENT APPLICATION

(11) **EP 2 127 641 A1**
(43) Date of publication of application: **02.12.2009**
(21) Application number: 08156932.9
(22) Date of filing: 26.05.2008
(51) Int. Cl.: A61K 9/14, A61K 31/137

(54) **Micronisable form of salmeterol xinafoate**

(71) Applicant: INKE, S.A., 08755 Bastellbisball - Barcelona (ES)
(72) Inventor: Dalmases Barjoan, Pere, E-08980, Sant Feliu de Llobregat - Barce (ES); Huguet Clotet, Juan, E-08970, Sant Joan Despí - Barcelona (ES)
(74) Representative: ABG Patentes, S.L.

(57) **Abstract**

The present invention relates to micronisable salmeterol xinafoate polymorph I characterized by a mean particle size of between 5 and 15 µm and a bulk density between 0.1 and 0.2 g/mL, to a method for its preparation and to its use in the preparation of micronised salmeterol xinafoate.

## Description

### FIELD OF THE INVENTION

The present invention relates to a novel micronisable form of salmeterol xinafoate, the process for its production and its use in the preparation of micronised salmeterol xinafoate.

### BACKGROUND

The phenethanolamine salmeterol xinafoate (I) is a long-active beta 2 adrenoreceptor agonist used in the treatment of bronchial asthma. Salmeterol xinafoate was described in GB 2140800 A together with its physiologically acceptable salts, specifically, its xinafoate salt. The drug is delivered by inhalation by means of either a metered-dose aerosol inhaler or a dry powder delivery device.

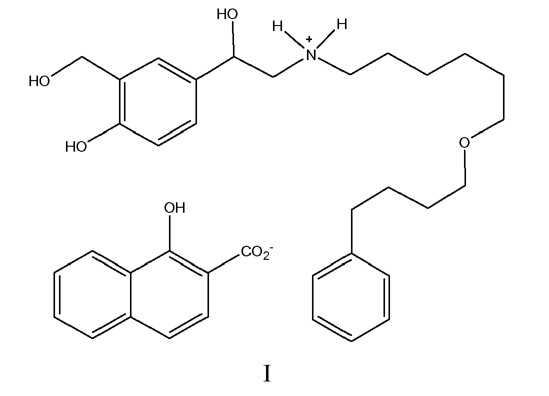

Before salmeterol xinafoate is formulated in the delivery device the drug substance is micronised to a size range about 2-5 µm to enable a suitable respirable fraction of drug particles to be delivered to the bronchial region of the lung. Micronisation is achieved using a fluid energy mill in which the crystalline powder is driven by air pressure into a cyclone.

Salmeterol xinafoate drug substance generated by a conventional crystallisation process has very poor powder flow properties, making it unsuitable for size reduction processes such as micronisation.

European patent EP 0 639 176 B1 provides a fast cooling crystallisation process which produces spherical agglomerates of small crystals which flow well and break up readily during micronisation.

European patent EP 0706507 B1 relates to a very rapid crystallization process using supercritical carbon dioxide which avoids micronisation of the final product. It also relates to salmeterol xinafoate in an easily fluidised crystalline form with a dynamic bulk density of less than 0.1 g.cm⁻³. According to the description, conventionally crystallised salmeterol xinafoate, when studied by differential scanning calorimetry (DSC), shows a transition between two forms (hereinafter "Polymorph I" and "Polymorph II") occurring between 120 and 140°C.

Henry H. Y. Tong et al in Pharmaceutical Research, 18, 6, 2001 describes the preparation of high pure polymorphs I and II of salmeterol xinafoate produced in a controlled one-step operation by the SEDS (Solution Enhanced Dispersion by Supercritical Fluids) technology.

However, some of the above-mentioned methods present several drawbacks derived from the use of supercritical fluids or mixtures of different boiling point solvents, which makes these procedures economically disadvantageous.

Therefore, it would be highly desirable to develop easy and economic methods to produce micronized salmeterol with good flowability properties, which overcome the drawbacks of the methods of the state of the art.

### BRIEF DESCRIPTION OF THE INVENTION

The authors of the present invention have surprisingly found a new micronisable form of salmeterol xinafoate polymorph I which shows good stability and appropriate physico-mechanical properties for its manipulation on industrial scale and final micronisation.

The novel micronisable form of salmeterol xinafoate is characterized for having a mean particle size lower than conventional crystallized micronisable salmeterol xinafoate, which facilitates the final micronisation of the product. Moreover, the inventors have found a process for preparing this novel micronisable salmeterol xinafoate which uses conventional crystallization methodologies and which does not require any special equipment.

Thus, a first aspect of the invention provides a micronisable salmeterol xinafoate polymorph I, characterized by a mean particle size between 5 and 15 µm and a bulk density from 0.1 to 0.2 g/mL.

A second aspect of the invention relates to a process for the preparation of the micronisable form of salmeterol xinafoate polymorph I of the invention, said process comprising:
a) seeding a solution of salmeterol xinafoate in an organic solvent with salmeterol xinafoate Polymorph I at a temperature approximately between 40 and 50°C;
b) subjecting the seeded solution of step a) to a stepwise profile cooling process.

This process has the advantage of being economical and easily industrialized.

A third aspect of the present invention refers to a micronisable salmeterol xinafoate polymorph I obtainable by a process as described above.

Finally, another aspect of the invention is directed to the use of such novel micronisable salmeterol xinafoate in the preparation of micronised salmeterol xinafoate.

### DETAILED DESCRIPTION OF THE INVENTION

As mentioned above, it is an object of the present invention to provide a novel micronisable form of salmeterol xinafoate polymorph I, characterized by a mean particle size between 5 and 15 µm and a bulk density from 0.1 to 0.2 g/mL.

By the term "micronisable form" it is understood a form which is easily broken down under micronising conditions, for example in a fluid-energy mill, to particles of a size suitable for use in a pharmaceutical dosage form to be delivered by inhalation or insufflation.

A feature derived from being micronisable is that the novel form is also free-flowing. This means that the form flows freely into a powder mill, for example a fluid energy powder mill, to allow its efficient particle size reduction by micronisation on an industrial scale. The physical characteristics of a material that determine its flow characteristics include its bulk density, cohesivity, specific surface area, particle size and shape and uniformity with respect to the particle size.

The bulk density of a powder includes the contribution of interparticulate void volume. Hence, the bulk density depends on both the density of powder particles and the space arrangement of particles in the powder bed. Bulk density is not an intrinsic property of a material, but it can change depending on how the material is handled. For this reason, the bulk density of powders is usually reported both as "freely settled" and "tapped" density (where the tapped density refers to the bulk density of the powder after a specified compaction process). European Pharmacopoeia 6.0, chapter 2.2.42 describes bulk and tapped density and its determination.

The novel micronisable form of the invention exhibits a bulk density between 0.1 and 0.2 g/mL, preferably between 0.12 and 0.17 g/mL.

When compared against conventionally crystallized micronisable salmeterol xinafoate, the micronisable form of the present invention exhibits a lower bulk density, which facilitates the preparation of micronized salmeterol xinafoate with well-balanced physico-mechanical properties in order to be administered by inhalation or insufflation.

Cohesiveness is a measure of the inter-particle forces (e.g. van der Waals forces). As particle size decreases, the inter-particle forces become stronger leading to an increase in cohesion. The increase in cohesion plays a dominant role in flow dynamics as it directly impacts the bulk flowabilibty of solid materials.

Specific surface area in the sense of the present invention means the BET-surface area, i.e. the surface area determined by nitrogen adsorption according to the method Brunauer, Emmet and Teller, S. Lowell and J.E. Shields, Powder Surface Area and Porosity, 1984, 2nd edition.

According to European Pharmacopoeia 6.0, chapter 2.9.36, compressibility is one of the standardized methods for characterizing powder flow.

The novel micronisable form of the invention has a mean particle size of from 5 to 15 µm, preferably of from 6 to 12 µm. This particle size is determined according to the monograph of European Pharmacopoeia 6.0, chapter 2.9.31. When compared against conventionally crystallized micronisable salmeterol xinafoate, the micronisable form of the present invention exhibits a lower mean particle size lower, which facilitates the final micronisation of the product.

A scanning electron micrograph (SEM) of some crystals of the novel micronisable salmeterol xinafoate shows that they are stable agglomerates of platelike crystals.

The present novel micronisable form may be prepared by any suitable method. However, in a particular embodiment, there is provided a process for the preparation of the micronisable form of salmeterol xinafoate polymorph I of the invention, said process comprising:
a) seeding a solution of salmeterol xinafoate in an organic solvent with salmeterol xinafoate Polymorph I at a temperature approximately between 40 and 50°C;
b) subjecting the seeded solution of step a) to a stepwise profile cooling process.

The authors of the present invention have found that the use of relatively low seeding temperatures, around 40-50°C, allows a subsequent rapid formation of the crystals of salmeterol xinafoate with an adequate mean particle size to be micronized. When the seeding temperature used is higher than about 55°C, the mean particle size of the resulting crystals reach over 20 µm, which makes difficult the final micronisation process.

In a preferred embodiment, the concentration of salmeterol xinafoate in the organic solvent solution before seeding is between 5 and 15 g/100 g solvent, more preferably between 8 and 10 g/100 g solvent.

Salmeterol xinafoate used as starting material for the purposes of this invention is prepared according to the process described in European patent EP 1132373 B1.

The term "stepwise profile cooling process" refers to a cooling process comprising two or more steps, wherein each step is carried out at a lower temperature than the previous one. In each cooling step the temperature is kept constant during a suitable time.

In a particular embodiment, the cooling process starts, after seeding, at a temperature between 40°C and 50°C, more preferably between 43-47°C and ends at a temperature between 5°C and -10°C, more preferably between 0 and -5°C. In a still more preferably embodiment, the cooling process starts at 45°C and ends at -5°C. This process comprises between 2 to 5 steps and the suitable time to keep the temperature constant in each step is between 30 min. to 2 hours.

In a particular embodiment, the process of the invention further comprises a previous step wherein salmeterol xinafoate is dissolved in the organic solvent at its boiling point and subsequently, said solution is subjected to a cooling step to reach a temperature between 40 and 50°C to carry out step a) of the process of the invention.

In a preferred embodiment, the organic solvent is an aliphatic ketone. Illustrative aliphatic ketones for use in the present invention include acetone, methyl isobutyl ketone, ethyl butyl ketone, dihexyl ketone, methyl ethyl ketone, diethyl ketone, diisobutyl ketone and mixtures thereof. Preferably, methyl ethyl ketone is used.

Unexpectedly, the process developed by the inventors provides polymorph I of salmeterol xinafoate with a mean particle between 5 and 15 µm, preferably between 6 and 12 µm and a bulk density from 01 to 0.2 g/mL, which is stable and suitable for manipulation and micronisation on an industrial scale.

Therefore, in a third aspect the present invention refers to a micronisable salmeterol xinafoate Polymorph I obtainable by a process as described above.

In a fourth aspect, the invention is directed to the use of the novel micronisable salmeterol xinafoate of the invention in the preparation of micronised salmeterol xinafoate. Preferably, the micronisable form of salmeterol xinafoate is micronised until the collected material has a particle size that is suitable for pharmaceutical dosage forms to be delivered by inhalation or insufflation. A suitable particle size for this use is less than 3 µm.

The micronization process may be carried out by any conventional micronizing techniques known by a skilled person, for example with a Trost or jet mill.

The following example is displayed to illustrate the micronisable form of the present invention and the process for its preparation. They do not intend to limit in any way the scope of the invention defined in the present description.

### EXAMPLES

### Example 1:

A suspension of 23 g of salmeterol xinafoate in 310 mL of methyl ethyl ketone is warmed to 70 °C under nitrogen. The solution obtained is cooled to 45 °C and seeded with 0.3 g of salmeterol xinafoate polimorph I. Cool further to 35 - 40 °C to get a consistent turbidity and keep on stirring for 30 min at that temperature. Cool down slowly to room temperature (20 - 25 °C) and stir for 1 hour. Cool down again to 0 °C and stir for at least 1 hour at that temperature. Filter the slurry under nitrogen pressure, wash with 40 mL of methyl ethyl ketone pre-cooled at 0 °C and dry the solid under vacuum at 30 °C. 21 g (91.3 %) of salmeterol xinafoate polimorph I are obtained with a mean particle size of 11.5 µm The physical properties of the novel micronisable salmeterol xinafoate obtained are:

| **Physical Property** | **Salmeterol xinafoate** |
|---|---|
| Bulk density (g·ml⁻¹) | 0.136 |
| Tapped density (g·ml⁻¹) | 0.256 |
| Compressibility (%) | 47.94 |
| BET Surface area (m²·g⁻¹) | 0.82 |
| Mean particle size (µm) | 11.5 |
| Cohesiveness | 74.8 |

## Claims

1. Micronisable salmeterol xinafoate polymorph I, **characterized by** a mean particle size between 5 and 15 µm and a bulk density from 0.1 and 0.2 g/mL.

2. Micronisable salmeterol xinafoate according to claim 1, wherein the mean particle size is comprised between 6 and 12 µm.

3. Micronisable salmeterol xinafoate according to claim 1 or 2 wherein the bulk density is comprised between 0.12 and 0.17 g/mL.

4. A process for preparing the micronisable salmeterol xinafoate as defined in any of claims 1 to 3, comprising:
a. seeding a solution of salmeterol xinafoate in an organic solvent with salmeterol xinafoate Polymorph I at a temperature approximately between 40 and 50°C;
b. subjecting the seeded solution of step a) to a stepwise profile cooling process.

5. The process according to claim 4 wherein the concentration of salmeterol xinafoate in the organic solvent solution before seeding is between 5 and 15 g/100 g solvent, more preferably between 8 and 10 g/100 g solvent.

6. The process according to claim 4 or 5 wherein the stepwise profile cooling process starts at a temperature between 40°C and 50°C and ends at a temperature between 5°C and -10°C.

7. The process according to claim 6 wherein the stepwise profile cooling process starts at a temperature between 43°C and 47°C and ends at a temperature between 0°C and -5°C.

8. The process according any one of claims 4 to 7 wherein the stepwise profile cooling process comprising between 2 to 5 steps.

9. The process according to claim 8 wherein the temperature is kept constant in each step during a time between 30 minutes and 2 hours.

10. The process according to any one of claims 4 to 9 which further comprises a previous step wherein salmeterol xinafoate is dissolved in the organic solvent at its boiling point and subsequently, said solution is subjected to a cooling step to reach a temperature between 40 and 50°C.

11. The process according to any one of claims 4 to 10 wherein the organic solvent is an aliphatic ketone selected from the group consisting of acetone, methyl isobutyl ketone, ethyl butyl ketone, dihexyl ketone, methyl ethyl ketone, diethyl ketone, diisobutyl ketone and mixtures thereof.

12. A micronisable salmeterol xinafoate Polymorph I obtainable by a process as defined in any one of claims 4 to 11.

13. Use of micronisable salmeterol xinafoate as defined in any one of claims 1 to 3 or 12 in the preparation of micronised salmeterol xinafoate.

14. Use according to claim 13 for the preparation of micronized salmeterol xinafoate having a particle size suitable for pharmaceutical dosage forms to be delivered by inhalation or insufflation.
